# EUROPEAN PATENT APPLICATION

(11) **EP 4 641 065 A1**
(43) Date of publication of application: **29.10.2025**
(21) Application number: 23906110.4
(22) Date of filing: 22.12.2023
(51) Int. Cl.: F16M 11/04

(54) **ROTATION LOCKING MECHANISM, ROTARY JOINT ASSEMBLY, AND MEDICAL DEVICE**

(30) Priority: 23.12.2022 CN 202223451332 U
(71) Applicant: Wuhan United Imaging Healthcare Co., Ltd., Wuhan, Hubei 430206 (CN)
(72) Inventor: SUN, Zengming, Wuhan, Hubei 430206 (CN)
(74) Representative: Murgitroyd & Company
(86) International application number: PCT/CN2023/141111
(87) International publication number: WO 2024/131955

(57) **Abstract**

A rotation locking mechanism includes a driving member, a locking assembly, and a locking counterpart. The driving member is configured to be slidably mounted within a rotary joint. The locking assembly is configured to be is at least partially mounted within the rotary joint and is movable relative to the rotary joint. The locking assembly is configured to form a positional interference with the driving member. The locking assembly is configured to move to a locking position in response to being pushed by the driving member, such that the locking assembly is limited by the locking counterpart. A rotary joint assembly includes a rotary joint and the rotation locking mechanism. A medical device includes a device body and the rotary joint assembly.

## Description

### CROSS-REFERENCE TO RELATED APPLICATIONS

This application claims priority to Chinese patent application No. 202223451332.0, entitled "ROTATION LOCKING MECHANISM, ROTARY JOINT ASSEMBLY, AND MEDICAL DEVICE", filed on December 23, 2022, the content of which is hereby incorporated by reference in its entirety.

### TECHNICAL FIELD

The present application belongs to the technical field of medical devices, particularly to a rotation locking mechanism, a rotary joint assembly, and a medical device.

### BACKGROUND

Rotary joints are important components in medical devices. The rotary joint in an unlocked state can drive equipment mounted on the rotary joint to rotate relative to the medical device, and in a locked state can lock the rotation relative to the medical device via a locking mechanism, thereby meeting the needs of using the equipment at different angles. Equipment commonly mounted on the rotary joints includes control panels, display screens, and the like.

In conventional medical devices, the locking mechanisms used to lock the rotary joints are typically located outside the rotary joints, which makes the rotary joint assemblies equipped with such locking mechanisms bulky and structurally complex, and occupy external space around the rotary joints, compromising the simplicity of the appearance and rendering operation inconvenient.

### SUMMARY

In view of the above, there is a need to provide a rotation locking mechanism, a rotary joint assembly, and a medical device.

A rotation locking mechanism, which is applied to a rotary joint, includes:
a driving member configured to be slidably mounted within the rotary joint;
a locking assembly configured to be at least partially mounted within the rotary joint and being movable relative to the rotary joint, the locking assembly being configured to form a positional interference with the driving member; and
a locking counterpart, the locking assembly being configured to move to a locking position in response to being pushed by the driving member, such that the locking assembly is limited by the locking counterpart.

In embodiments of the present application, by arranging the driving member and the locking assembly for locking the rotary joint inside the rotary joint, and exploiting the push action of the moving driving member on the locking assembly to control the engagement between the locking assembly and the locking counterpart, the purpose of locking the rotary joint can be achieved. This structure not only simplifies operation but also provides the rotary joint assembly, including the rotation locking mechanism and the rotary joint, with a clean appearance, reducing the space occupied by the rotary joint assembly in the mounted device e.g., a medical device, further meeting the application requirements of the device.

In some embodiments, the locking assembly includes a guide sleeve and a locking member, and the locking member is slidably mounted to the guide sleeve; the guide sleeve at least partially protrudes toward the driving member to form the positional interference with the driving member, and the guide sleeve is configured to push the locking member to partially extend out from the rotary joint in response to the guide sleeve being pushed by the driving member, such that the locking member is engaged with and limited by the locking counterpart.

In some embodiments, a length direction of the guide sleeve extends along a radial direction of the rotary joint, the locking member is disposed in the guide sleeve and is slidable along the radial direction of the rotary joint.

In some embodiments, the locking assembly further includes a limiting pin, and the locking member is slidably connected to the guide sleeve via the limiting pin.

In some embodiments, the locking assembly further includes a first elastic member, the first elastic member is disposed between the locking member and the guide sleeve, and the guide sleeve is capable of elastically pushing the locking member through the first elastic member.

In some embodiments, the locking member is provided with a slot, the limiting pin extends through the slot and is fixed on the guide sleeve, and the limiting pin is slidable relative to the locking member between two ends of the slot along the radial direction of the rotary joint.

In some embodiments, the rotation locking mechanism further includes a fixing seat, and the fixing seat is configured to be fixed on the rotary joint to at least partially retain the locking assembly within the rotary joint.

In some embodiments, the rotation locking mechanism further includes a second elastic member, and the second elastic member is configured to make the locking assembly elastically abut against the driving member.

In some embodiments, a side surface of the driving member includes a track surface, the locking assembly includes a pushing engagement head located at an end of the locking assembly, and the track surface is engaged with the pushing engagement head to push the locking assembly to move relative to the rotary joint.

In some embodiments, the side surface of the driving member defines a first limiting groove and a second limiting groove, a surface of the first limiting groove and a surface of the second limiting groove cooperatively form the track surface; on a condition that the driving member is located at a first position, the pushing engagement head is located in the first limiting groove; on a condition that the driving member is located at a second position, the pushing engagement head is located in the second limiting groove.

In some embodiments, a depth of the first limiting groove is less than a depth of the second limiting groove.

In some embodiments, the depth of the second limiting groove allows the locking member to disengage from limitation of the locking counterpart.

In some embodiments, a surface of the pushing engagement head, the surface of the first limiting groove, and the surface of the second limiting groove are all arc-shaped surfaces, and the pushing engagement head is capable of being engaged with the surface of the first limiting groove and the surface of the second limiting groove, respectively.

In some embodiments, the locking counterpart is provided with at least one locking hole, the at least one locking hole is arranged corresponding to the locking assembly, and the locking assembly is capable of being inserted into the at least one locking hole.

In some embodiments, the at least one locking hole includes a plurality of locking holes, and the plurality of locking holes are arranged along a circumferential direction of the rotary joint and spaced apart from each other.

In some embodiments, the locking counterpart is of a ring structure, the locking counterpart comprises including a cylindrical sidewall disposed on an outer periphery thereof, the rotary joint is adapted to be arranged at a center of the ring structure, and a distance between the cylindrical sidewall and the rotary joint allows the locking assembly to move between the cylindrical sidewall and the rotary joint.

In some embodiments, the rotation locking mechanism further includes a lever, the lever is mounted on the driving member and partially extends out from the rotary joint, and under a drive of the lever, the driving member is controlled to move relative to the rotary joint.

A rotary joint assembly includes a rotary joint and the above-described rotation locking mechanism.

In some embodiments, the rotary joint has an accommodating groove configured to accommodate the driving member, and the rotary joint has a through hole allowing the locking assembly to extend therethrough, a size of the accommodating groove is larger than a size of the driving member, such that the driving member is slidable within the rotary joint; the through hole is in communication with the accommodating groove.

A medical device includes a device body and the above-described rotary joint assembly, wherein the locking counterpart is fixed relative to the device body.

### BRIEF DESCRIPTION OF THE DRAWINGS

To more clearly illustrate the embodiments of the present application or the technical solutions in the prior art, the following briefly introduces the drawings required for describing the embodiments or the prior art. Obviously, the drawings in the following description are only some embodiments of the present application. For those skilled in the art, other drawings can be obtained based on these drawings without creative effort.
FIG. 1 is a structural schematic partial view of a rotary joint assembly mounted on a medical device according to an embodiment of the present application.
FIG. 2 is a cross-sectional view of FIG. 1, taken along an axial direction, wherein a locking assembly is limited by a locking counterpart.
FIG. 3 is an enlarged view of section P in FIG. 2.
FIG. 4 is a structural schematic view of a driving member according to an embodiment of the present application.
FIG. 5 is a cross-sectional partial view of a rotary joint assembly, taken along an axial direction, according to an embodiment of the present application, wherein a driving member is located at a second position, and a locking assembly is located at a non-locking position.
FIG. 6 is a cross-sectional view of a rotary joint assembly, taken along a radial direction, according to an embodiment of the present application, wherein a locking counterpart is a ring structure with one locking hole.
FIG. 7 is a cross-sectional view of a rotary joint assembly, taken along a radial direction, according to another embodiment of the present application, wherein a locking counterpart is a ring structure with multiple locking holes.
FIG. 8 is an enlarged view of section Q in FIG. 7.
FIG. 9 is a cross-sectional partial view of a rotary joint assembly, taken along an axial direction, according to an embodiment of the present application, wherein a driving member is located at a first position, and a locking member is not limited by a locking assembly.
FIG. 10 is a cross-sectional view of a rotary joint assembly, taken along an axial direction, according to another embodiment of the present application, wherein a locking counterpart is located inside a rotary joint.
FIG. 11 is a cross-sectional view of a rotary joint assembly, taken along a radial direction, according to another embodiment of the present application, wherein a locking counterpart is located inside a rotary joint.
FIG. 12 is a cross-sectional partial view of a rotary joint assembly, taken along a radial direction, according to another embodiment of the present application, wherein a driving member is slidable along a circumferential direction.
FIG. 13 is a cross-sectional view of a rotary joint assembly, taken along a radial direction, according to another embodiment of the present application, wherein a driving member is slidable along a circumferential direction, and a locking counterpart is located inside a rotary joint.

### DETAILED DESCRIPTION

The following will clearly and completely describe the technical solutions in the embodiments of the present application with reference to the accompanying drawings. Obviously, the described embodiments are only some of, rather than all, embodiments of the present application. Based on the embodiments of the present application, all other embodiments obtained by those of ordinary skill in the art without creative effort shall fall within the protection scope of the present application.

In the present application, an element, when being referred to as being "connected" to another element, may be directly fixed or mounted to the other element or via an intermediate element. An element, when being referred to as being "fixed" or "mounted" to another element, may be directly connected to the other element or via an intermediate element. Such terms as "vertical", "horizontal", "up", "down", "left", "right" and the like used herein are for illustrative purposes only and are not meant to be the only ways for implementing the present application.

In the present application, unless otherwise clearly specified and defined, the terms "installed", "connected", "coupled", "fixed" and the like should be interpreted broadly. For example, one element may be fixedly connected, detachably connected, or integrated to the other element, may be mechanically connected or electrically connected to the other element, may be directly connected to the other element or connected to the other element via an intermediate element, unless otherwise specifically defined. For those of ordinary skill in the art, the specific meaning of the above-mentioned terms in the present application can be understood based on the context.

In the present application, the terms "first" and "second" are merely used for descriptive purposes, and should not be construed as indicating or implying relative importance or implying the quantity or order of the described technical features. Therefore, the feature modified by "first" or "second" may explicitly or implicitly be at least one such feature. In the description of the present application, "a plurality of" means at least two, such as two, three, etc., unless otherwise expressly and specifically defined.

Unless defined otherwise, all technical and scientific terms used in the present application have the same meaning as commonly understood by those skilled in the art of the present application. The terms used in the specification of the present application are for the purpose of describing exemplary examples only and are not intended to limit the present application. The term "and/or" as used herein refers to any and all combinations of one or more listed items.

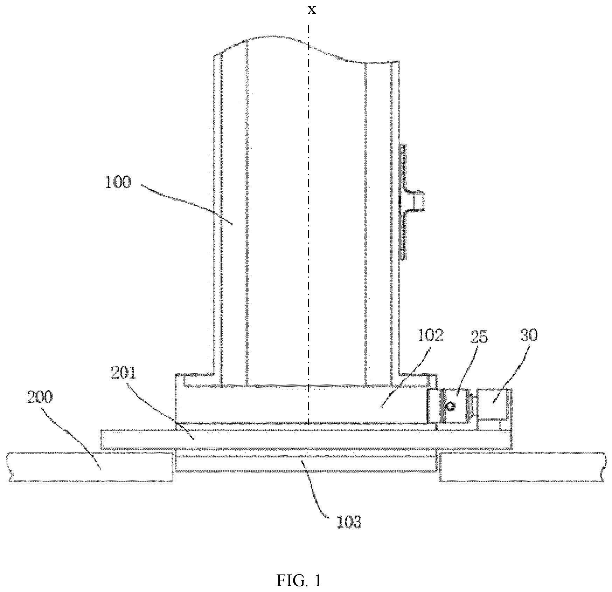

Referring to FIG. 1, a first aspect of the present application provides a rotation locking mechanism, applied to a rotary joint 100, for locking the rotation of the rotary joint 100 relative to a device body 200. The rotary joint 100 can be part of an apparatus or a separate component configured to be connected to the apparatus. In some embodiments, the rotary joint 100 is configured to drive the apparatus to rotate around an axis of the rotary joint 100, e.g., axis x shown in FIG. 1, thereby changing the orientation of the apparatus.

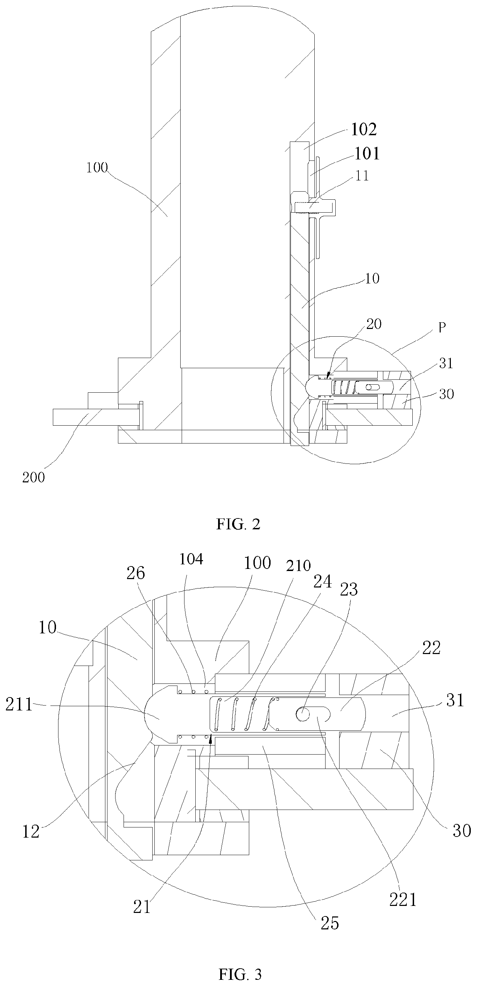

Referring to FIG. 2, the rotation locking mechanism includes a driving member 10, a locking assembly 20, and a locking counterpart 30. The driving member 10 is configured to control the movement of the locking assembly 20 by forming a positional interference with the locking assembly 20. The locking assembly 20 is configured to move between a non-locking position and a locking position. At the locking position, the locking assembly 20 is limited by the locking counterpart 30 to lock the axial rotation of the rotary joint 100 relative to the locking counterpart 30. At the non-locking position, the axial rotation of the rotary joint 100 is unlocked.

The locking counterpart 30 can be fixed relative to the device body 200, thereby locking or unlocking the axial rotation of the rotary joint 100 relative to the device body 200. The rotation locking mechanism has a simple structure and is easy to operate.

Specifically, the locking assembly 20 is fixed relative to the rotary joint 100 in the circumferential direction of the rotary joint 100, and the locking assembly 20 is movable relative to the rotary joint 100 in the radial direction of the rotary joint 100 between the locking position and the non-locking position in response to sliding of the driving member 10 between a first position and a second position. At the locking position, the locking assembly 20 is circumferentially limited by the locking counterpart 30, thereby locking the rotation of the rotary joint 100. In the non-locking position, the locking assembly 20 is separated from the locking counterpart 30, thus allowing the rotary joint 100 to rotate around the axis.

Referring to FIG. 3, in some embodiments, the driving member 10 is configured to be slidably mounted inside the rotary joint 100. The locking assembly 20 is configured to be at least partially mounted inside the rotary joint 100 and movable relative to the rotary joint 100. A positional interference can be formed between the locking assembly 20 and the driving member 10. The locking assembly 20 is configured to move to the locking position in response to being pushed by the driving member 10, such that the locking assembly 20 is limited by the locking counterpart 30 to lock the rotation of the rotary joint 100 relative to the locking counterpart 30. When the locking counterpart 30 is fixed to the device body 200, the locking assembly 20 locks the rotation of the rotary joint 100 relative to the device body 200.

It can be understood that by arranging the driving member 10 and the locking assembly 20 for locking the rotary joint 100 inside the rotary joint 100, and exploiting the push action of the moving driving member 10 on the locking assembly 20 to control the engagement between the locking assembly 20 and the locking counterpart 30, the purpose of locking the rotary joint 100 can be achieved. This structure not only simplifies operation but also provides the rotary joint assembly, including the rotation locking mechanism and the rotary joint 100, with a simple appearance, reducing the space occupied by the rotary joint assembly in the mounted device e.g., a medical device, further meeting the application requirements of the device.

The driving member 10 is slidable between the first position and the second position inside the rotary joint 100. Correspondingly, the rotary joint 100, e.g., its sidewall, has an accommodating groove 102 for accommodating the driving member 10. The size of the accommodating groove 102 allows the driving member 10 to slide between the first position and the second position.

In some embodiments, the driving member 10 is movable along the axial direction (i.e., the axis x) relative to the rotary joint 100, while the locking assembly 20 is movable along the radial direction relative to the rotary joint 100, and the locking counterpart 30 can be fixedly mounted on the device body 200. In the axial direction of the rotary joint 100, an axial size of the accommodating groove 102 is larger than an axial size of the driving member 10, thus allowing the driving member 10 to slide axially inside the accommodating groove 102. In these embodiments, since the driving member 10 only needs to slide along the axial direction, the driving member 10 can be a guide post mounted inside the rotary joint 100, and the length direction of the driving member 10 is aligned with the axial direction of the rotary joint 100.

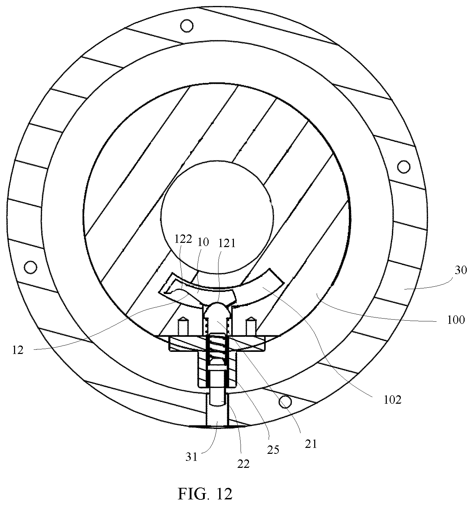

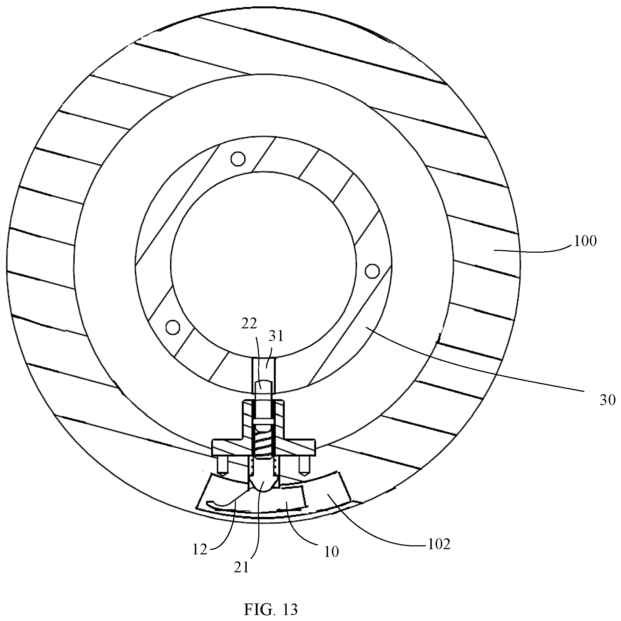

Referring to FIGs. 12 and 13, in some other embodiments, the driving member 10 is movable along the circumferential direction relative to the rotary joint 100, i.e., rotatable around the axis x relative to the rotary joint 100. Correspondingly, a circumferential size of the accommodating groove 102 of the rotary joint 100 is larger than a circumferential size of the driving member 10, thus allowing the driving member 10 to slide circumferentially inside the accommodating groove 102. Specifically, the cross-sections of the driving member 10 and the accommodating groove 102 perpendicular to the axis x are both arc-shaped, and the center of the arcs coincide with the axis x of the rotary joint 100. Specifically, the driving member 10 can be an arc-shaped sliding piece. A lengthwise edge of the driving member 10 can be curved circumferentially around the rotary joint 100, forming an arc shape with its center coinciding with the axis x of the rotary joint 100. Alternatively, the length direction of the driving member 10 can be aligned with the axial direction of the rotary joint 100, and a widthwise edge of the driving member 10 can be curved circumferentially around the rotary joint 100, forming an arc shape with its center coinciding with the axis x of the rotary joint 100.

It can be understood that the sliding direction of the driving member 10 is not limited to the axial or circumferential direction of the rotary joint 100, as long as the driving member 10 is movable between the first position and the second position.

In some embodiments, the rotation locking mechanism further includes a lever 11. The lever 11 is mounted on the driving member 10 and partially extends out from the rotary joint 100. Under the control of the lever 11, the driving member 10 can move with the lever 11 relative to the rotary joint 100. In some embodiments, the lever 11 extends radially out from the rotary joint 100.

The rotary joint 100 can be provided with a strip slot 101 allowing the lever 11 to extend out. The strip slot 101 has sufficient size in the sliding direction of the driving member 10, e.g., the axial or circumferential direction of the rotary joint 100, such that the lever 11 can slide relative to the rotary joint 100 in the strip slot 101 and drive the driving member 10 to slide synchronously in the accommodating groove 102. In some embodiments, the strip slot 101 is located on the surface of the rotary joint 100 and is in communication with the accommodating groove 102.

Corresponding to the locking assembly 20 of the rotation locking mechanism, the rotary joint 100, e.g., its sidewall, has a through hole 104 allowing the locking assembly 20 to extend therethrough. The through hole 104 is in communication with the accommodating groove 102, and the locking assembly 20 extends through the through hole 104 to form the positional interference with the driving member 10 inside the rotary joint 100.

The length direction of the through hole 104 is aligned with the radial direction of the rotary joint 100. The locking assembly 20 is arranged in the through hole 104 of the rotary joint 100 and is movable in the through hole 104 along the radial direction of the rotary joint 100, while being fixed relative to the rotary joint 100 along the circumferential direction of the rotary joint 100, thus being capable of rotating around the axis synchronously with the rotary joint 100.

The locking assembly 20 can be of a columnar structure. The length direction of the locking assembly 20 can be aligned with the radial direction of the rotary joint 100. In some embodiments, the length of the locking assembly 20 is fixed, e.g., the locking assembly 20 is of a one-piece columnar structure. In another embodiment, the length of the locking assembly 20 is variable, such as being able to shorten in response to external space compression and elongate when external space permits. With a length-variable locking assembly 20, before manipulating the driving member 10 to push the locking assembly 20, the user does not need to strictly rotate the rotary joint 100 to the locking angle to align the locking assembly 20 with the locking hole 31 of the locking counterpart 30. Instead, the user can slide the driving member 10 to push the locking assembly 20 at any position. When the locking assembly 20 is not aligned with the locking hole 31 of the locking counterpart 30, the length of the locking assembly 20 will be compressed. When the locking assembly 20 is rotated to be aligned with the locking hole 31, the locking assembly 20 will elongate and extend into the locking hole 31 to achieve positional limitation through the locking counterpart 30. Therefore, the length-variable locking assembly 20 can provide a certain fault tolerance, making operation more convenient.

In some embodiments, the length-variable locking assembly 20 includes a first component and a second component, and the first and second components are connected end-to-end in the length direction of the locking assembly 20. In some embodiments, one of the first and second components can be at least partially inserted into the other, allowing the overall length of the locking assembly 20 to vary. In other embodiments, a first elastic member 24 can be further arranged between the first and second components, thus allowing the overall length of the locking assembly 20 to vary.

In some embodiments of the length-variable locking assembly 20, the first component is a guide sleeve 21, and the second component is a locking member 22. The locking member 22 is slidably mounted to the guide sleeve 21. Specifically, the guide sleeve 21 can define a guide sleeve slot 210 extending in the length direction of the guide sleeve 21, and the locking member 22 can be arranged in the guide sleeve slot 210. The guide sleeve 21 at least partially protrudes toward the driving member 10 to form the positional interference with the driving member 10, and the guide sleeve 21 is configured to push the locking member 22 to the locking position in response to the guide sleeve 21 being pushed by the driving member 10, such that the locking member 22 is engaged with and limited by the locking counterpart 30.

In some other embodiments, the first component is of a rod-shaped structure, and the second component is of a sleeve-shaped structure. The rod-shaped structure at least partially protrudes toward the driving member 10 to form the positional interference with the driving member 10, and the rod-shaped structure is configured to push the sleeve-shaped structure to the locking position in response to the rod-shaped structure being pushed by the driving member 10, such that the sleeve-shaped structure is engaged with and limited by the locking counterpart 30.

Taking the guide sleeve 21 and the locking member 22 as an example for further description, the guide sleeve 21 and the locking member 22 cooperatively form the locking assembly 20, allowing the driving member 10 to push the locking member 22 to partially extend out from the rotary joint 100 through the guide sleeve 21 to be engaged with the locking counterpart 30 for position limitation, thereby locking the rotary joint 100 while simplifying the structure. Specifically, the length direction of the guide sleeve 21 is aligned with the radial direction of the rotary joint 100, and the locking member 22 is arranged in the guide sleeve 21, allowing the locking member 22 to slide along the radial direction of the rotary joint 100. The guide sleeve 21 protrudes toward the driving member 10 along the radial direction of the rotary joint 100, so as to contact and form the positional interference with the side surface of the driving member 10. When the driving member 10 moves relative to the rotary joint 100 along the axial direction of the rotary joint 100, the end of the guide sleeve 21 is in contact with different positions on the side surface of the driving member 10 to form the positional interference, so that in response to the push of the driving member 10, the locking member 22 is pushed to partially extend out from the rotary joint 100 and form positional interference with the locking counterpart 30. The embodiment with the rod-shaped structure and sleeve-shaped structure has similar functions and effects, which will not be repeated.

In some embodiments, the locking assembly 20 further includes a limiting pin 23 and/or a first elastic member 24. The locking member 22 is slidably connected to the guide sleeve 21 via the limiting pin 23 to prevent the locking member 22 from completely detaching from the guide sleeve 21. The first elastic member 24 is disposed between the locking member 22 and the guide sleeve 21, so that the guide sleeve 21 is capable of elastically pushing the locking member 22 through the first elastic member 24, thereby making the overall length of the locking assembly 20 variable. In an embodiment, the first elastic member 24 is a spring; in another embodiment, the first elastic member 24 is a rubber sleeve. The first elastic member 24 can be non-fixed or fixed to the guide sleeve 21 and the locking member 22, respectively. In some embodiments, one end of the first elastic member 24 can be fixed to the bottom of the guide sleeve slot 210, and the other end of the first elastic member 24 can be fixed to the end of the limiting pin 23.

It can be understood that the sliding connection between the locking member 22 and the guide sleeve 21 via the limiting pin 23 can simplify the structure and ensure consistent movement direction of the locking member 22 in the guide sleeve 21. Since the locking member 22 and the guide sleeve 21 are slidably connected, their relative positions can change, thus allowing the overall length of the locking assembly 20 to vary. Since the first elastic member 24 is disposed between the limiting pin 23 and the guide sleeve 21, the guide sleeve 21 can elastically push the locking member 22 through the first elastic member 24, making the length variation of the locking assembly 20 elastic. The extended length of the locking member 22 can elastically vary through the first elastic member 24 when extending out from the rotary joint 100, thus facilitating engagement with the locking counterpart 30.

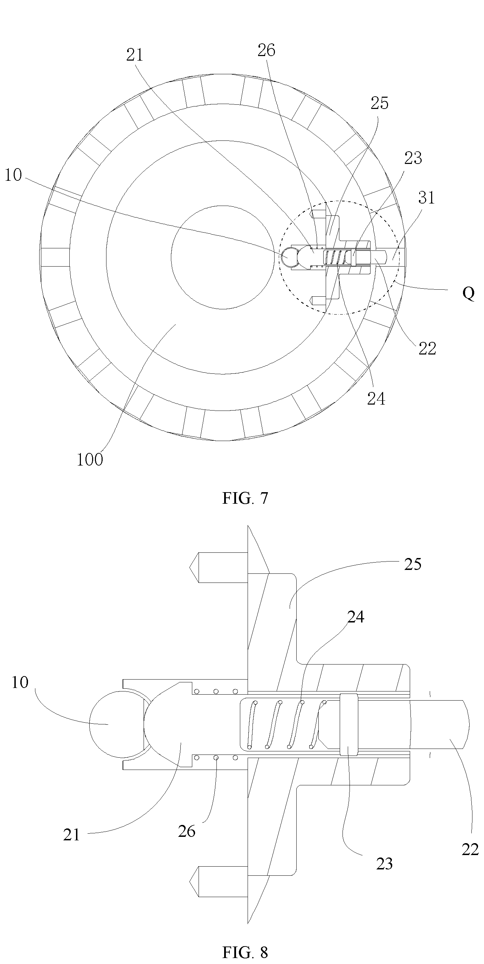

Specifically, referring to FIGs. 3 and 8, the guide sleeve 21 has a pin hole, and the locking member 22 has a slot 221. The limiting pin 23 can extend through the pin hole and the slot 221 and be fixed on the guide sleeve 21, allowing the moving guide sleeve 21 to drive the limiting pin 23 to move in the slot 221 of the locking member 22. Thus, the limiting pin 23 and the slot 221 cooperate to limit the maximum sliding distance of the locking member 22 in the guide sleeve 21, and the maximum sliding distance is equal to the length of the slot 221 along the radial direction of the rotary joint 100. Specifically, the slot 221 extends in the length direction of the guide sleeve 21, i.e., the radial direction of the rotary joint 100, allowing the locking member 22 to slide along the length direction of the guide sleeve 21, i.e., the radial direction of the rotary joint 100, between the two ends of the slot 221 relative to the locking member 22.

In some embodiments, the rotation locking mechanism further includes a fixing seat 25. The fixing seat 25 is fixedly mounted on the rotary joint 100 and can rotate with the rotary joint 100. The fixing seat 25 is configured to more reliably retain the guide sleeve 21 within the rotary joint 100. In some embodiments, the fixing seat 25 is a separate component fixed to the rotary joint 100 via fasteners such as screws, bolts, or clips. In other embodiments, the fixing seat 25 is integrated with the rotary joint 100 or is part of the rotary joint 100.

In some embodiments, the rotation locking mechanism further includes a driving counterpart, such as a second elastic member 26 or an equivalent structure such as a magnetic component. The driving counterpart is configured to make the guide sleeve 21 elastically abut against the side surface of the driving member 10, so as to drive the locking assembly 20 to move to the non-locking position when the driving member 10 slides back to the second position, thereby cooperating with the driving member 10 to release the limitation of the locking counterpart 30 on the locking assembly 20. The following description takes the second elastic member 26 as an example. The second elastic member 26 can be disposed between the guide sleeve 21 and the fixing seat 25. The structure of the second elastic member 26 can be the same as or different from that of the first elastic member 24. The second elastic member 26 is sleeved outside the guide sleeve 21, with one end thereof fixed to the end of the guide sleeve 21, and the other end thereof fixed to the end of the fixing seat 25 or the inner wall of the through hole 104 of the rotary joint 100.

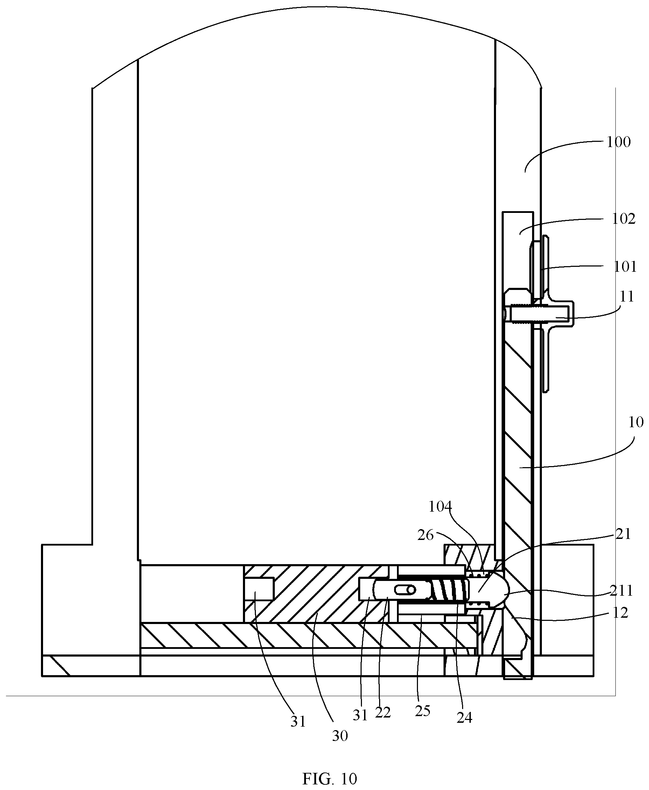

Referring to FIGs. 3, 10, 12, and 13, in some embodiments, the side surface of the driving member 10 includes a track surface 12. The locking assembly 20 includes a pushing engagement head 211 located at an end of the locking assembly 20. The track surface 12 is capable of engaging with the pushing engagement head 211 to push the guide sleeve 21 to move relative to the rotary joint 100. In other words, the driving member 10 drives the guide sleeve 21 to move through the engagement between the track surface 12 and the pushing engagement head 211. Specifically, the side surface of the driving member 10 defines one or more limiting grooves locally, forming the track surface 12.

In some embodiments, the side surface of the driving member 10 defines a first limiting groove 121 and a second limiting groove 122. The surface of the first limiting groove 121 and the surface of the second limiting groove 122 together form the track surface 12. The pushing engagement head 211 can move into the first limiting groove 121 or the second limiting groove 122.

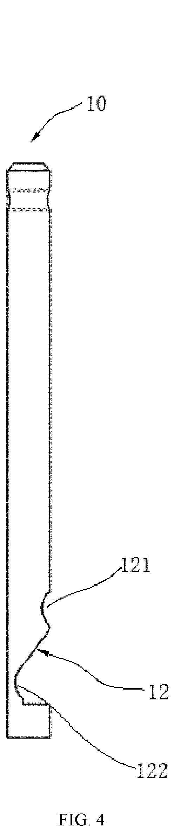

Referring to FIG. 4, in some embodiments, the driving member 10 rotates relative to the rotary joint 100 around the axial direction of the rotary joint 100, and correspondingly the first limiting groove 121 and the second limiting groove 122 are arranged along the axial direction of the rotary joint 100.

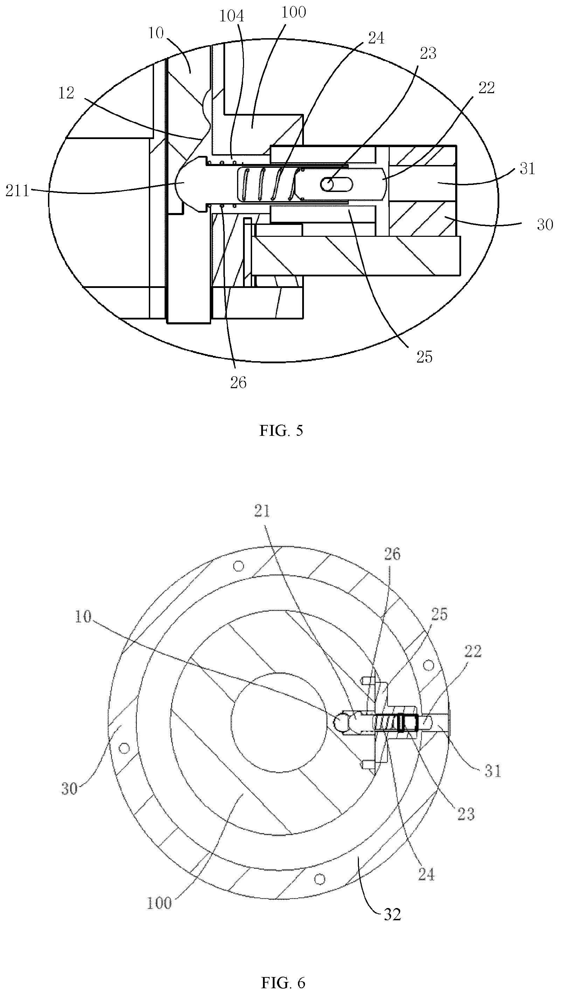

Referring to FIGs. 3 and 5, when the driving member 10 is located at the first position, the pushing engagement head 211 is directed to the first limiting groove 121 and enters the first limiting groove 121 under the action of the second elastic member 26, i.e., the locking assembly 20 moves to the locking position. When the driving member 10 is located at the second position, the pushing engagement head 211 is directed to the second limiting groove 122 and enters the second limiting groove 122 under the action of the second elastic member 26, i.e., the locking assembly 20 moves to the non-locking position. The second elastic member 26 is more compressed when the locking assembly 20 is located at the locking position than at the non-locking position, and thus can push the guide sleeve 21 into the second limiting groove 122 when the driving member 10 moves to the second position. The first limiting groove 121 is shallower, only serving to provide a positional limitation between the driving member 10 and the guide sleeve 21. When the pushing engagement head 211 of the guide sleeve 21 enters the first limiting groove 121, the end of the locking member 22 can still form the positional limitation with the locking counterpart 30, thus preventing the rotary joint 100 from rotating. The second limiting groove 122 is deeper, not only providing the positional relationship between the driving member 10 and the guide sleeve 21, but also allowing the locking member 22 to be disengaged from the limitation of the locking counterpart 30, enabling the rotary joint 100 to rotate. In other words, when the driving member 10 moves inside the rotary joint 100, the first limiting groove 121 and the second limiting groove 122 can be adopted to engage with the pushing engagement head 211 of the guide sleeve 21 for positioning, thereby simplifying the structure and facilitating the control of locking or unlocking the rotary joint 100 during operation of the rotation locking mechanism.

Referring to FIGs. 12 and 13, in some other embodiments, the driving member 10 moves along the circumferential direction relative to the rotary joint 100, and correspondingly the first limiting groove 121 and the second limiting groove 122 are arranged along the circumferential direction of the rotary joint 100. The interaction between the locking assembly 20 (e.g., the guide sleeve 21, the locking member 22, the pushing engagement head 211, etc.) and the driving member 10 moving circumferentially is similar to that with the driving member 10 moving axially, and will not be repeated herein.

**In** some embodiments, the track surface 12 of the driving member 10 further includes a relatively smooth transition surface. The transition surface is connected between the first limiting groove 121 and the second limiting groove 122, allowing the driving member 10 to easily switch between the first limiting groove 121 and the second limiting groove 122.

In some embodiments, the surface of the pushing engagement head 211, the surface of the first limiting groove 121, and the surface of the second limiting groove 122 are all arc-shaped surfaces, and the pushing engagement head 211 can cooperate with the surfaces of the first limiting groove 121 and the second limiting groove 122, respectively, facilitating the guide sleeve 21 to switch between the first limiting groove 121 and the second limiting groove 122 when the driving member 10 moves.

It can be understood that the surface or shape of the pushing engagement head 211, the first limiting groove 121, and the second limiting groove 122 only needs to allow the pushing engagement head 211 to overcome a resistance threshold when moving between the first limiting groove 121 and the second limiting groove 122. Therefore, the surface of the pushing engagement head 211, the surface of the first limiting groove 121, and the surface of the second limiting groove 122 are not limited to arc-shaped surfaces. For example, at least part of the surface of the pushing engagement head 211, the surface of the first limiting groove 121, and the surface of the second limiting groove 122 are flat surfaces, such as being formed by connecting multiple flat segments at different angles.

In some embodiments, the locking counterpart 30 is provided with a locking hole 31. The locking hole 31 is arranged corresponding to the locking assembly 20. When the driving member 10 is located at the first position, the locking assembly 20, e.g., the locking member 22, can be inserted into the locking hole 31 to prevent the rotation of the rotary joint 100, specifically realizing the engagement and positional limitation between the locking counterpart 30 and the locking assembly 20.

The locking counterpart 30 can be fixedly mounted on the device body 200, and can be disposed outside or inside the rotary joint 100.

In some embodiments, the locking counterpart 30 is disposed outside the rotary joint 100, e.g., as shown in FIGs. 1 to 3, 5 to 7, 9, and 12. Correspondingly, the locking assembly 20 is configured to extend out from the rotary joint 100 in response to the push of the driving member 10, thereby forming the positional limitation with the locking counterpart 30 disposed outside the rotary joint 100. The side surface with the track surface 12 of the driving member 10 faces outward. Correspondingly, the pushing engagement head 211 of the locking assembly 20 for engaging with the track surface 12 faces inward. The through hole 104 of the rotary joint 100 can be defined on the outer sidewall of the rotary joint 100, so as to allow the locking assembly 20 to extend outward from the rotary joint 100.

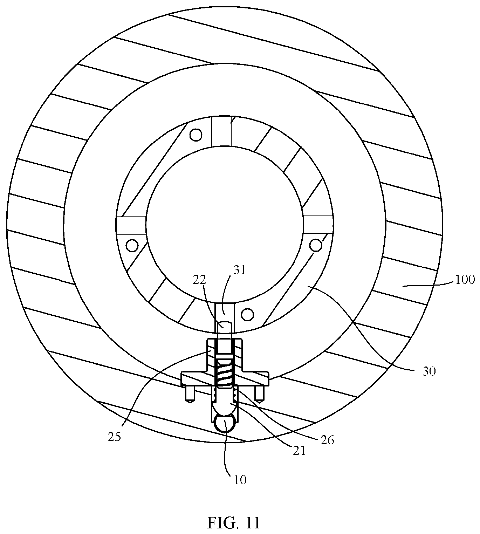

Referring to FIGs. 11 and 13, in some other embodiments, the locking counterpart 30 is disposed inside the rotary joint 100. Correspondingly, the locking assembly 20 is configured to extend toward the locking counterpart 30 inside the rotary joint 100 in response to the push of the driving member 10, thereby forming the positional limitation with the locking counterpart 30. Specifically, the locking counterpart 30 can be disposed at the center of the rotary joint 100. The side surface with the track surface 12 of the driving member 10 faces inward. Correspondingly, the pushing engagement head 211 of the locking assembly 20 for engaging with the track surface 12 faces outward. The through hole 104 of the rotary joint 100 can be defined on the inner sidewall of the rotary joint 100, so as to allow the locking assembly 20 to extend toward the center of the rotary joint 100.

In some embodiments, the locking counterpart 30 is disposed corresponding to a specific circumferential position of the rotary joint 100. When the rotary joint 100 rotates to this position, by sliding the driving member 10, the locking member 22 can be engaged with and limited by the locking counterpart 30, thereby restricting the rotation of the rotary joint 100.

Referring to FIG. 6, in some other embodiments, the locking counterpart 30 is a ring structure disposed around the periphery of the rotary joint 100. The rotary joint 100 is disposed at the center of the ring structure. The ring structure includes a cylindrical sidewall 32.

The cylindrical sidewall 32 is spaced apart from the rotary joint 100 by a certain distance. When the locking assembly 20 protrudes from the outer or inner sidewall of the rotary joint 100, this distance can be relatively large. When the locking assembly 20 is entirely arranged within the sidewall of the rotary joint 100, this distance can be relatively small. In other words, as long as the locking counterpart 30 is disposed at a position that allows the rotary joint 100 and the locking assembly 20 in the non-locking position to rotate around the axis, and allows the locking assembly 20 in the locking position to extend into the locking hole 31 of the locking counterpart 30 to be circumferentially limited by the locking counterpart 30, the locking counterpart 30 can be arranged at any location.

By cooperating the cylindrical sidewall 32 with the length-variable locking assembly 20, the user does not need to strictly align the locking member 22 with the locking hole 31 of the locking counterpart 30 before locking the rotary joint 100. Instead, the user can first slide the driving member 10 to the first position when the rotary joint 100 is rotated to any position, and then continue rotating the rotary joint 100 until the locking member 22 is aligned with the locking hole 31 of the locking counterpart 30, thereby locking the rotary joint 100.

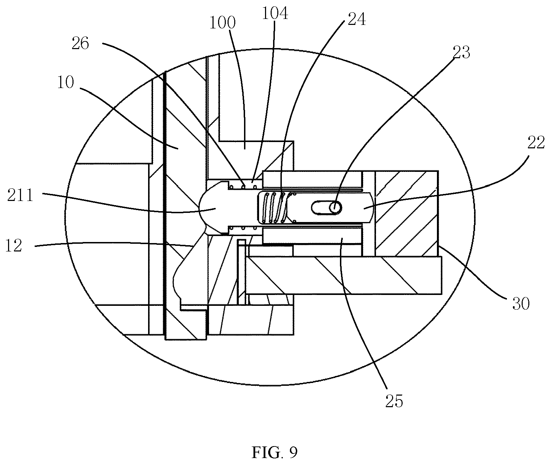

Specifically, referring to FIG. 9, when the driving member 10 moves from the second position to the first position, the guide sleeve 21 moves outward along the radial direction of the rotary joint 100. If the locking member 22 is not yet aligned with the locking hole 31, the locking member 22 will abut against the cylindrical sidewall 32. Since the locking member 22 has the slot 221, the limiting pin 23 can slide in the slot 221, allowing the locking member 22 to move toward the inside of the guide sleeve 21, i.e., toward the rotary joint 100 along the radial direction of the rotary joint 100, thereby shortening the overall length of the locking assembly 20. At this time, the first elastic member 24 is compressed. When the rotary joint 100 continues rotating until the locking member 22 is aligned with the locking hole 31 of the locking counterpart 30, the locking member 22 is pushed by the first elastic member 24 to move toward the outside of the guide sleeve 21, i.e., away from the rotary joint 100 along the radial direction of the rotary joint 100, and enters the locking hole 31, thereby locking the rotary joint 100, during which the overall length of the locking assembly 20 increases. Since the limiting pin 23 is fixed in the guide sleeve 21, the relative position of the locking member 22 in the guide sleeve 21 is limited by the length of the slot 221, thereby preventing the locking member 22 from completely detaching from the guide sleeve 21.

Referring to FIG. 7, in some embodiments, the quantity of the locking holes 31 is multiple. The multiple locking holes 31 are arranged along the circumferential direction of the rotary joint 100 and spaced apart from each other. Specifically, the multiple locking holes 31 can be disposed on the cylindrical sidewall 32, allowing the rotation locking mechanism to lock the rotary joint 100 at different rotation angles.

A second aspect of the present application provides a rotary joint assembly, including the rotary joint 100 and the rotation locking mechanism described in the first aspect.

A third aspect of the present application provides a medical device, including the device body 200, the rotary joint 100, and the rotation locking mechanism described above. The rotary joint 100 is rotatably mounted on the device body 200 via the rotation locking mechanism and can be locked relative to the device body 200 under the action of the rotation locking mechanism.

In some embodiments, the rotary joint 100 includes a revolute pair 102 and a connecting base plate 103. The rotary joint 100 can be rotatably mounted on a mounting plate 201 of the device body 200 via the revolute pair 102 and the connecting base plate 103, specifically realizing the rotatably connection of the rotary joint 100 with the device body 200.

In the rotation locking mechanism, rotary joint assembly, and medical device of the embodiments of the present application, by arranging the driving member 10 and the locking assembly 20 for locking the rotary joint inside the rotary joint 100, and exploiting the push action of the moving driving member 10 on the locking assembly 20 to control the engagement between the locking assembly 20 and the locking counterpart 30, the purpose of locking the rotary joint 100 can be achieved. This structure not only simplifies operation but also provides the rotary joint assembly with a simple appearance, reducing the space occupied by the rotary joint assembly in the medical device, further meeting the application requirements of the medical device.

The technical features of the above-mentioned embodiments can be combined arbitrarily. In order to make the description concise, not all possible combinations of the technical features are described in the embodiments. However, as long as there is no contradiction in the combination of these technical features, the combinations should be considered as in the scope of the present disclosure.

The above-described embodiments are only several implementations of the present disclosure, and the descriptions are relatively specific and detailed, but they should not be construed as limiting the scope of the present disclosure. It should be understood by those of ordinary skill in the art that various modifications and improvements can be made without departing from the concept of the present disclosure, and all fall within the protection scope of the present disclosure. Therefore, the patent protection of the present disclosure shall be defined by the appended claims.

## Claims

1. A rotation locking mechanism, applied to a rotary joint (100), the rotation locking mechanism comprising:
a driving member (10) configured to be slidably mounted within the rotary joint (100);
a locking assembly (20) configured to be at least partially mounted within the rotary joint (100) and being movable relative to the rotary joint (100), the locking assembly (20) being configured to form a positional interference with the driving member (10); and
a locking counterpart (30), the locking assembly (20) being configured to move to a locking position in response to being pushed by the driving member (10), such that the locking assembly (20) is limited by the locking counterpart (30).

2. The rotation locking mechanism according to claim 1, wherein the locking assembly (20) comprises a guide sleeve (21) and a locking member (22), and the locking member (22) is slidably mounted to the guide sleeve (21);
wherein the guide sleeve (21) at least partially protrudes toward the driving member (10) to form the positional interference with the driving member (10), and the guide sleeve (21) is configured to push the locking member (22) to partially extend out from the rotary joint (100) in response to the guide sleeve (21) being pushed by the driving member (10), such that the locking member (22) is engaged with and limited by the locking counterpart (30).

3. The rotation locking mechanism according to claim 2, wherein a length direction of the guide sleeve (21) extends along a radial direction of the rotary joint (100), the locking member (22) is disposed in the guide sleeve (21) and is slidable along the radial direction of the rotary joint (100).

4. The rotation locking mechanism according to claim 2 or 3, wherein the locking assembly (20) further comprises a limiting pin (23), and the locking member (22) is slidably connected to the guide sleeve (21) via the limiting pin (23).

5. The rotation locking mechanism according to claim 4, wherein the locking assembly (20) further comprises a first elastic member (24), the first elastic member (24) is disposed between the locking member (22) and the guide sleeve (21), and the guide sleeve (21) is capable of elastically pushing the locking member (22) through the first elastic member (24).

6. The rotation locking mechanism according to claim 4 or 5, wherein the locking member (22) is provided with a slot (221), the limiting pin (23) extends through the slot (221) and is fixed on the guide sleeve (21), and the limiting pin (23) is slidable relative to the locking member (22) between two ends of the slot (221) along the radial direction of the rotary joint (100).

7. The rotation locking mechanism according to any one of claims 1 to 6, further comprising a fixing seat (25), wherein the fixing seat (25) is configured to be fixed on the rotary joint (100) to at least partially retain the locking assembly (20) within the rotary joint (100).

8. The rotation locking mechanism according to any one of claims 1 to 7, further comprising a second elastic member (26), wherein the second elastic member (26) is configured to make the locking assembly (20) elastically abut against the driving member (10).

9. The rotation locking mechanism according to any one of claims 1 to 8, wherein a side surface of the driving member (10) comprises a track surface (12), the locking assembly (20) comprises a pushing engagement head (211) located at an end of the locking assembly (20), and the track surface (12) is engaged with the pushing engagement head (211) to push the locking assembly (20) to move relative to the rotary joint (100).

10. The rotation locking mechanism according to claim 9, wherein the side surface of the driving member (10) defines a first limiting groove (121) and a second limiting groove (122), a surface of the first limiting groove (121) and a surface of the second limiting groove (122) cooperatively form the track surface (12);
on a condition that the driving member (10) is located at a first position, the pushing engagement head (211) is located in the first limiting groove (121); on a condition that the driving member (10) is located at a second position, the pushing engagement head (211) is located in the second limiting groove (122).

11. The rotation locking mechanism according to claim 10, wherein a depth of the first limiting groove (121) is less than a depth of the second limiting groove (122).

12. The rotation locking mechanism according to claim 11, wherein the depth of the second limiting groove (122) allows the locking member (22) to disengage from limitation of the locking counterpart (30).

13. The rotation locking mechanism according to any one of claims 10 to 12, wherein a surface of the pushing engagement head (211), the surface of the first limiting groove (121), and the surface of the second limiting groove (122) are all arc-shaped surfaces, and the pushing engagement head (211) is capable of being engaged with the surface of the first limiting groove (121) and the surface of the second limiting groove (122), respectively.

14. The rotation locking mechanism according to any one of claims 1 to 13, wherein the locking counterpart (30) is provided with at least one locking hole (31), the at least one locking hole (31) is arranged corresponding to the locking assembly (20), and the locking assembly (20) is capable of being inserted into the at least one locking hole (31).

15. The rotation locking mechanism according to claim 14, wherein the at least one locking hole (31) comprises a plurality of locking holes (31), and the plurality of locking holes (31) are arranged along a circumferential direction of the rotary joint (100) and spaced apart from each other.

16. The rotation locking mechanism according to any one of claims 1 to 15, wherein the locking counterpart (30) is of a ring structure, the locking counterpart (30) comprises a cylindrical sidewall (32) disposed on an outer periphery thereof, the rotary joint (100) is adapted to be disposed at a center of the ring structure, and a distance between the cylindrical sidewall (32) and the rotary joint (100) allows the locking assembly (20) to move between the cylindrical sidewall (32) and the rotary joint (100).

17. The rotation locking mechanism according to any one of claims 1 to 16, further comprising a lever (11), wherein the lever (11) is mounted on the driving member (10) and partially extends out from the rotary joint (100), and under a drive of the lever (11), the driving member (10) is controlled to move relative to the rotary joint (100).

18. A rotary joint assembly, comprising a rotary joint (100) and the rotation locking mechanism according to any one of claims 1 to 17.

19. The rotary joint assembly according to claim 18, wherein the rotary joint (100) has an accommodating groove (102) configured to accommodate the driving member (10), and the rotary joint (100) has a through hole (104) allowing the locking assembly (20) to extend therethrough, a size of the accommodating groove (102) is larger than a size of the driving member (10), such that the driving member (10) is slidable within the rotary joint (100); the through hole (104) is in communication with the accommodating groove (102).

20. A medical device, comprising a device body (200) and the rotary joint assembly according to any one of claims 18 to 19, wherein the locking counterpart (30) is fixed relative to the device body (200).
